# EUROPÄISCHE PATENTANMELDUNG

(11) **EP 1 577 376 A2**
(43) Veröffentlichungstag der Anmeldung: **21.09.2005**
(21) Anmeldenummer: 05002349.8
(22) Anmeldetag: 04.02.2005
(51) Int. Cl.: C12M 1/107

(54) **Fermenter einer Biogasanlage mit einer Rühreinrichtung**

(30) Priorität: 16.03.2004 DE 202004004101 U
(71) Anmelder: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen

(57) **Zusammenfassung**

Die Erfindung betrifft einen Fermenter einer Biogasanlage mit wenigstens einer Rühreinrichtung, mit einer mittels eines Rührmotors drehangetriebenen und weit in den Fermenterbehälter ragenden Rührerwelle und wenigstens einem an der Rührerwelle mitdrehend angebrachten Rührpaddel, welches unter die Flüssigkeitsoberfläche der vergärbaren Fermenterflüssigkeit mit Biomasse eintauchbar ist. Erfindungsgemäß ist der Rührmotor an einem oberen Rührerwellenende angeschlossen und dieses ragt in der Funktionsstellung über die Flüssigkeitsoberfläche und aus einer Montageöffnung in einer Fermenterdeckenwand oder aus einer Montageöffnung im oberen Bereich einer Fermenterseitenwand heraus. Das wenigstens eine Rührpaddel ist um eine etwa senkrecht zur Rührerwellenachse liegende, mit der Rührerwelle verbundene Achse klappbar verbunden, und einerseits in eine von der Rührerwelle abragende Funktionsstellung aufklappbar und mittels einer Fixiereinrichtung fixierbar und andererseits für eine Durchmesserverkleinerung der Anordnung in eine Ausziehstellung für die Rührerwelle an diese anklappbar. Die Größe der Montageöffnung ist dem verkleinerten Durchmesser der Anordnung aus der Rührerwelle und des daran angeklappten wenigstens einen Rührpaddels angepasst und die Anordnung ist in dieser Ausziehstellung für Wartungs- und/oder Reparaturzwecke aus dem Fermenter durch die Montageöffnung herausziehbar und demontierbar.

## Beschreibung

Die Erfindung betrifft einen Fermenter einer Biogasanlage mit einer Rühreinrichtung nach dem Oberbegriff des Anspruchs 1.

In Biogasanlagen läuft ein Fermentationsprozess ab, bei dem organische Stoffe, wie beispielsweise Wirtschaftsdünger aus der Landwirtschaft (Rindergülle, Rinder-Festmist, Schweinegülle, Schweine-Festmist, Hühnergülle, Hühner-Trockenkot) und/oder landwirtschaftliche Reststoffe (Grasschnitt-Rübenblätter, Silagen) und/oder Reststoffe aus der Agroindustrie oder verwandten Industrien (Biertreber, Obstreste, Gemüsereste, Rapsschrot, Getreideabputz, Schlempen, Melasse) als Biomasse vergast werden. Die hierbei entstehenden Gase sammeln sich in einem oberen Fermenterbehälterbereich eines Fermenterbehälters und können direkt zur Energieerzeugung verwendet werden, z. B. als Heizgas zur Stromerzeugung in nachgeschalteten Brennkraftmaschinen mit Elektrogeneratoren. Zur Fermentation werden im Fermenterbehälter die organischen Stoffe mit Flüssigkeit versetzt und Mikroorganismen, wie z. B. Hefen, Bakterien, etc. zugeführt, so dass der Fermentations- bzw. der Vergasungsprozess unter aeroben oder anaeroben Bedingungen ablaufen kann.

Ein Problem bei derartigen allgemein bekannten Fermentationsprozessen ist, dass die Biomasse, insbesondere Biomasse-Feststoffe in der Fermenterflüssigkeit regelmäßig nicht gleichmäßig verteilt sind, da sie z. B. aufschwimmen und sich im Bereich der Flüssigkeitsoberfläche ansammeln, was z. B. der Fall ist, wenn das spezifische Gewicht der Biomasse geringer ist als dasjenige der Fermenterflüssigkeit, z. B. Wasser. Andererseits kann aber auch das Problem bestehen, dass Biomasse, insbesondere Biomasse-Feststoffe mit einem höheren spezifischen Gewicht als die Fermenterflüssigkeit auf den Fermenterbehälterboden absinken und sich dort als Sinkschichten ansammeln. Auch eine derartige Ansammlung von Biomasse bewirkt eine ungleichmäßige Biomasseverteilung in der Fermenterflüssigkeit, was sich insgesamt nachteilig auf den Wirkungsgrad des Fermentationsprozesses auswirkt.

Für eine möglichst gleichmäßige Verteilung der Biomasse in der Fermenterflüssigkeit zur Steigerung des Wirkungsgrades sind bereits Rühreinrichtungen bekannt (DE 197 32 168 C1). Die Rühreinrichtung ist hier durch ein Tauchmotorrührgerät gebildet, das an einem vertikal im Behälter angeordneten Aggregatträger höhenverstellbar gehalten ist, wobei ein Tauchmotor eine etwa horizontal liegende Rührerwelle mit einem Propellerrührer antreibt. Eine derartige Rühreinrichtung mit einem an einer Rührerwelle angeordneten Propellerrührer führt im wesentlichen zu einer Vermischung der Biomasse und der Fermentationsflüssigkeit in einem lokal begrenzten Bereich, da insbesondere der Propellerdurchmesser im Vergleich zur Fermenterbehältergröße relativ klein ist. In entfernteren Fermentationsflüssigkeitsbereichen kann es daher trotz der Verwendung eines oder mehrerer Tauchmotorrührgeräte zu einer Entmischung zwischen der festeren Biomasse und der Fermenterflüssigkeit und zu einer unerwünschten Phasentrennung mit der Ausbildung von unerwünschten Schwimm- oder Sinkschichten kommen.

Weiter ist es bekannt (DE 197 14 342 C2) ein solches Tauchmotorrührgerät zu Wartungs- und/oder Reparaturzwecken mittels einer Seilwinde in einen als Dom ausgeführten Serviceschacht durch eine Wartungsöffnung in der Fermenterdeckewand nach oben heraus zu ziehen. Dies ist vorteilhaft möglich, ohne dass der Flüssigkeitsspiegel im Fermenter abgesenkt werden muss und damit der Fermentationsprozess gestört wird. Die Abmessungen eines solchen Tauchrührgeräts sind relativ gering, so dass der Serviceschacht und die Wartungsöffnung ebenfalls entsprechend relativ geringe Abmessungen aufweisen können.

Weiter ist ein gattungsgemäßer Fermenter einer Biogasanlage mit wenigstens einer Rühreinrichtung bekannt (DE 199 28 212 A1), mit einer mittels eines Rührmotors drehangetriebenen und weit in den Fermenterbehälter ragenden Rührerwelle und wenigstens einem an der Rührerwelle mitdrehend angebrachten Rührpaddel, welches unter die Flüssigkeitsoberfläche der vergärbaren Fermenterflüssigkeit mit Biomasse eintauchbar ist. Konkret besteht hier der Fermenterbehälter aus einem zylinderrohrförmigen, liegenden Tank mit einer zentralen, horizontal liegenden Rührerwelle, an der axial und radial zueinander versetzt eine Mehrzahl von Rührpaddeln angeordnet sind. Bei einer solchen Anordnung eines Fermenters mit einer Paddelrühreinrichtung kann diese zu Reparatur- und Wartungsarbeiten ersichtlich nicht aus dem Fermenterbehälter einfach entnommen werden. Zu solchen Arbeiten muss der Fermenterbehälter entleert werden und ein Werker muss durch ein Mannloch in den Behälter einsteigen mit den bekannten Problemen zur Arbeitssicherheit, da Biogase schädlich und explosibel sind. Für größere Reparaturarbeiten an der Paddelrühreinrichtung muss zu deren Entnahme ein Kopfteil des Fermenterbehälters demontiert werden. Diese Gegebenheiten führen daher bei solchen Fermenter- und Rühreranordnungen zu erheblichen Problemen und Erschwernissen.

Aufgabe der Erfindung ist es, einen gattungsgemäßen Fermenter einer Biogasanlage mit wenigstens einer Rühreinrichtung so weiterzubilden, dass bei einfachem Aufbau des Fermenterbehälters die Rühreinrichtung ohne Absenkung des Flüssigkeitsspiegels repariert und/oder gewartet werden kann.

Diese Aufgabe wird mit den Merkmalen des Anspruchs 1 gelöst.

Gemäß Anspruch 1 ist der Rührmotor an einem oberen Rührerwellenende angeschlossen, wobei dieses in Funktionsstellung über die Flüssigkeitsoberfläche und aus einer Montageöffnung in einer Fermenterdeckenwand oder aus einer Montageöffnung im oberen Bereich einer Fermenterseitenwand herausragt. Somit steht die Rührerwelle bei diesen Anordnungen entweder vertikal oder ragt schräg nach unten geneigt in den Fermenterbehälter hinein.

Für eine geeignete Rühr- und Mischwirkung ist es erforderlich, das oder die Rührpaddel relativ lange auszuführen, so dass sie weit von der Rührerwelle abragen und mit einem großen Rührdurchmesser mit relativ kleiner Drehzahl die Fermenterflüssigkeit umwälzen. Aus Gründen der Behälterstatik, der Arbeitssicherheit und der Handhabung kann die Montageöffnung nicht so groß ausgebildet werden, dass die Rührerwelle mit weit abstehenden Rührpaddeln für Wartungs- und Reparaturzwecke - ähnlich wie das eingangs erwähnte Tauchmotorrührgerät - aus dem Fermenterbehälter herausgezogen werden kann. Um ein Herausziehen ohne Absenkung des Flüssigkeitsspiegels dennoch zu ermöglichen werden folgende weitere Maßnahmen vorgeschlagen:

Das wenigstens eine Rührpaddel ist um eine etwa senkrecht zur Rührerwellenachse liegende, mit der Rührerwelle verbundene Achse klappbar verbunden. Dabei ist das Rührpaddel einerseits in eine von der Rührerwelle abragende Funktionsstellung aufklappbar und mittels einer Fixiereinrichtung fixierbar und andererseits für eine Durchmesserverkleinerung der Anordnung in eine Ausziehstellung für die Rührerwelle an diese anklappbar. Grundsätzlich kann dieses Anklappen an die Rührerwelle nach unten oder nach oben erfolgen, wobei es üblicherweise günstiger ist nach Lösung der Fixiereinrichtung das Rührpaddel durch sein Gewicht nach unten klappen zu lassen.

Die Größe der Montageöffnung ist dem verkleinerten Durchmesser der Anordnung aus der Rührerwelle und des daran angeklappten wenigstens einen Rührpaddels angepasst, so dass die Montageöffnung relativ klein in zulässiger Weise ausgeführt sein kann, ohne dass dadurch eine hinsichtlich der Behälterstatistik und/oder der Handhabung und/oder der Arbeitssicherheit ungünstig große Öffnung in der Behälterdeckenwand oder Behälterseitenwand erforderlich ist. Die Anordnung kann in der Ausziehstellung mit einem oder mehreren angeklappten Rührpaddeln für Wartungs- und Reparaturzwecke bequem aus dem Fermenterbehälter durch die Montageöffnung herausgezogen werden. Das Anheben und Herausziehen erfolgt zweckmäßig mit einer mobilen Hubeinrichtung, wie beispielsweise einem Kranfahrzeug.

Für ein gutes Rühr- und Mischergebnis ist es nach Anspruch 2 vorteilhaft, wenn mehrere Rührpaddel gegebenenfalls auch mit unterschiedlicher Rührpaddellänge axial und/oder radial gegeneinander versetzt an der Rührerwelle angeordnet sind. Gut bewährt hat sich eine Anordnung nach Anspruch 3, wo jeweils zwei von mehreren Rührpaddeln auf gleicher Höhe gegenüberliegend an der Rührerwelle angeordnet sind.

Weiter hat sich gezeigt, dass ein gutes Rühr- und Mischergebnis erreicht wird, wenn nach Anspruch 4 ein oder mehrere Rührpaddel als längliche Paddelplatten ausgebildet sind mit einem in der Funktionsstellung bestimmten Anstellwinkel der Platten gegenüber der Rührerwellenachse. Dabei sollen die Paddelplattenflächen weitgehend senkrecht ausgerichtet sein. Die Oberflächen der Paddelplatten sowie deren Form können zudem profiliert und dadurch an die jeweiligen Rührgegebenheiten angepasst werden.

Für einen störungsfreien Drehbetrieb der Rührerwelle und eine Abstützung der auftretenden Gegenkräfte ist es nach Anspruch 5 zweckmäßig das untere Rührerwellenende mittels einer, ein Herausziehen ermöglichenden lösbaren Steckverbindung ortsfest am Fermenterboden festzulegen. Die Steckverbindung kann für eine gezielte Einführung als Fangtasche mit trichterförmigen Einführschrägen oder als inneres ortsfestes Führungsrohr ausgebildet sein, über die die Rührerwelle als Hohlwelle aufgesteckt ist.

Die Steckverbindung selbst kann dabei als Drehlager für die Rührerwelle verwendet werden. In einer verbesserten Ausführung nach Anspruch 6 ist versetzt zum unteren Rührerwellenende jedoch in dessen Nähe ein Drehlager als Bestandteil der herausziehbaren Rührerwelle angebracht. Das Rührerwellenende ist somit ein nicht mitdrehendes Fußteil als Bestandteil der Steckverbindung, welches hinsichtlich einer Wartung oder Reparatur unkritisch ist. Das Drehlager wird zu Wartungs- und/oder Reparturzwecken bequem mit der Rührerwelle aus dem Fermenter herausgezogen.

Gemäß Anspruch 7 ist es zweckmäßig bei einem Fermenter mit einer stabilen Decke, insbesondere einer Betondecke die Rührerwelle vertikal und bei einem Fermenter mit Foliendach schräg nach unten anzubringen.

Vorteilhaft kann gemäß Anspruch 8 auch hier ein an sich bekannter Serviceschacht verwendet werden, der entweder über einer Montageöffnung einer stabilen Fermenterdecke oder seitlich anschließend an eine Montageöffnung in einer Seitenwand angebracht ist. Alternativ oder zusätzlich kann das obere Rührerwellenende in der Montageöffnung auch durch eine gasdicht abschließende Vorrichtung, wie z. B. eine Manschette oder eine Tauchtasse geführt sein.

In einer relativ einfachen und preiswerten Ausführung nach Anspruch 9 kann die Fixiereinrichtung für die Halterung des wenigstens einen Rührpaddels in der Funktionsstellung aus einer lösbaren Verriegelungsverbindung bestehen, wobei der lösbare Riegel als Bolzen und/oder Verschraubung und/oder Schiebehülse ausgebildet sein kann. Für eine Einstellung und Anpassung des Aufklappwinkels in der Funktionsstellung an unterschiedliche Gegebenheiten kann die Verriegelungsverbindung nach Anspruch 10 zwei oder mehrere Eingriffpositionen aufweisen, denen jeweils ein bestimmter Klappwinkel des Rührpaddels in der Funktionsstellung zugeordnet ist.

Bei dieser Art von Fixiereinrichtung mit einer lösbaren Verriegelungsverbindung wird die Rührerwelle jeweils so weit herausgezogen, bis ein noch ausgeklapptes Rührpaddel von innen her in den Bereich der (kleineren) Montageöffnung gelangt. In dieser Position wird dann durch die Montageöffnung hindurch von einem Werker die im Bereich der zugänglichen Rührerwelle angeordnete Fixiereinrichtung bequem gelöst und dieses Rührpaddel abgeklappt, wonach die Anordnung mit reduziertem Durchmesser weiter, gegebenenfalls bis zu einem weiteren noch ausgeklappten Rührpaddel herausgezogen werden kann. Nach einer entsprechenden Lösung aller Fixiereinrichtungen und nach einem Abklappen aller Rührpaddel kann dann die Rührervvelle schrittweise insgesamt herausgezogen werden.

In einer alternativen Weiterbildung nach Anspruch 11 wird eine Fixiereinrichtung als betätigbare Stelleinrichtung vorgeschlagen, mit der von außerhalb des Fermenters, insbesondere im Bereich des oberen Rührerwellenendes der Klappwinkel des wenigstens einen Rührpaddels oder mehrerer Rührpaddel von der Funktionsstellung bis zur angeklappten Ausziehstellung verstellbar ist. Damit ist eine Art Fernverstellung gegeben, so dass gegebenenfalls die Klappwinkel eines oder mehrerer Rührpaddel ohne Herausziehen der Rührerwelle verändert und an unterschiedliche Rührgegebenheiten und zu Optimierzwecken anpassbar sind oder die Rührpaddel in die Ausziehstellung anklappbar sind.

Eine solche Stelleinrichtung kann nach Anspruch 12 für eine mechanische Verstellung einfach aus wenigstens einer Schubstange hergestellt sein, die entlang und innerhalb oder außerhalb der Rührerwelle verläuft. Eine solche Schubstange ist mit wenigstens einem Rührpaddel, vorzugsweise gekoppelt mit mehreren oder allen vorhandenen Rührpaddeln, stellbeweglich verbunden und am oberen Rührerwellenendenbereich von Hand oder durch einen Schubstangenstellmotor betätigbar sowie in einer jeweils bestimmten Stellposition entsprechend einem bestimmten Klappwinkel festlegbar.

In einer aufwendigeren Ausführung nach Anspruch 13 kann eine solche Stelleinrichtung anstelle eine Schubstangensteuerung auch aus einem oder mehreren unmittelbar an der Rührerwelle angeordneten und eingetauchten sowie mit einem oder mehreren Rührpaddeln verbundenen hydraulischen und/oder elektrischen Rührpaddel-Stellmotoren ausgeführt sein. Damit ist auch eine Fernverstellung des Klappwinkels angepasst an Prozessabläufe von einem zentralen Steuergerät aus möglich.

Ähnlich wie der Klappwinkel des einen oder mehrerer Rührpaddel bezüglich der Rührerwellenachse fernverstellbar ausgeführt sein kann, ist es auch möglich nach Anspruch 14 den Anstellwinkel schwenkbar gelagerter Paddelplatten fernbedienbar für unterschiedliche Rührgegebenheiten anzupassen und zu optimieren. Auch hierzu kann eine mechanische Verstellung über Schubstangen, Seilzüge etc. oder über jeweils zugeordnete Stellmotoren erfolgen.

Es hat sich in Versuchen gezeigt, dass eine besonders intensive Durchmischung und gleichmäßige Verteilung von Biomasse in der Fermenterflüssigkeit in Verbindung mit einer Effektivitätssteigerung des Biogasprozesses erreichbar ist, wenn gemäß Anspruch 15 wenigstens eine vorstehende Rühreinrichtung mit Rührpaddeln zusammen mit wenigstens einem vorzugsweise mehreren der eingangs erwähnten höhenverstellbaren Tauchmotorrührwerken eingesetzt wird.

### Anhand einer Zeichnung wird die Erfindung näher erläutert.

Es zeigen:
- Fig. 1: eine schematische Schnittdarstellung durch eine erste Ausführungsform eines Fermenters einer Biogasanlage mit einer Rühreinrichtung, wobei eine Rührwelle vertikal ausgerichtet ist,
- Fig. 2: eine schematische Schnittdarstellung durch eine zweite Ausführungsform eines Fermenters einer Biogasanlage mit einer Rühreinrichtung, wobei eine Rührwelle schräg ausgerichtet angeordnet ist,
- Fig. 3: eine schematische Detailvergrößerung eines Teilbereichs der Rührwelle von Fig. 2 mit einer Verriegelungsverbindung zwischen einem Rührpaddel und der Rührwelle, und
- Fig. 4: eine schematische Detailvergrößerung eines Teilbereichs der Rührwelle von Fig. 2 mit einer Stelleinrichtung für ein Klappen des Rührpaddels.

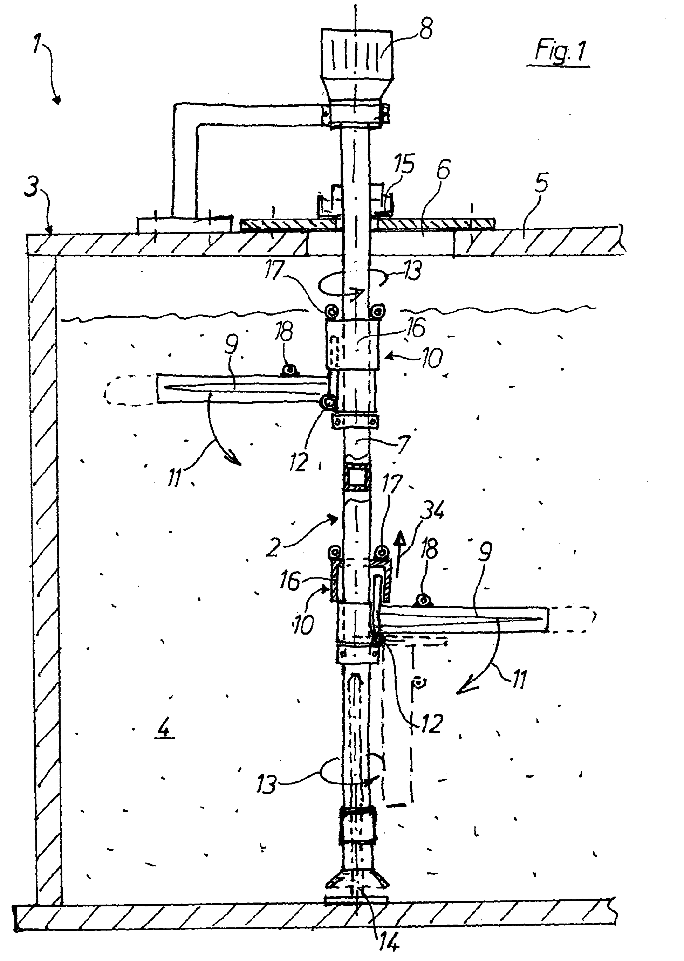

In Fig. 1 ist schematisch eine Schnittdarstellung durch einen Fermenter 1 einer Biogasanlage mit einer Rühreinrichtung 2 gezeigt. Der Fermenter 1 weist einen Fermenterbehälter 3 auf, in dem die vergärbare Fermenterflüssigkeit 4 mit Biomasse aufgenommen ist, wobei der Fermenterbehälter 3 in der in Fig. 1 gewählten Ansicht nur teilweise dargestellt ist. Der Fermenterbehälter 3 weist eine Betondecke 5 als Fermenterdeckenwand auf, in der eine Montageöffnung 6 angeordnet ist. Die Rühreinrichtung 2 ist so durch die Montageöffnung 6 in den Fermenterbehälter 3 hineingesteckt, dass eine Rührwelle 7 weit in den Fermenterbehälter 3 hineinragt und ein Drehantrieb 8 der Rührwelle als Bestandteil der Rühreinrichtung 2 oberhalb der Montageöffnung 6 und somit außerhalb des Fermenterbehälters 3 angeordnet ist. An der Rührwelle 7 sind zwei Rührpaddel 9 angeordnet, die bei in den Fermenterbehälter 3 eingebauter Rühreinrichtung 2 unterhalb der Flüssigkeitsoberfläche der Fermenterflüssigkeit 4 angeordnet sind. Die Rührpaddel 9 sind als längliche Paddelplatten ausgebildet, die in der in Fig. 1 mit durchgezogenen Linien dargestellten Funktionsstellung einen bestimmten Anstellwinkel gegenüber der Rührwelle 7 bzw. der Rührwellenachse aufweisen.

Die Rührpaddel 9 sind mittels einer Fixiereinrichtung 10 in der Funktionsstellung gehalten, wobei bei einer Lösung der Fixiereinrichtung 10 ein Abklappen in eine Ausziehstellung in Pfeilrichtung eines Pfeiles 11 zur Rührwelle 7 hin möglich ist. Die Ausziehstellung ist beim unteren Rührpaddel 9 mit strichliert langen Strichen eingezeichnet. Für eine funktionssichere Klappung können die Rührpaddel 9 dabei um eine in etwa senkrecht zur Rührwelle 7 liegende mit der Rührwelle 7 verbundene Klappachse 12 geklappt werden.

Beim Betrieb des Fermenters 1 der Biogasanlage wird die in der Fermenterflüssigkeit 4 aufgenommene Biomasse für ein Verhindern eines etwaigen Absinkens bzw. Aufschwimmens der Biomasse innerhalb der Fermenterflüssigkeit 4 mittels der Rühreinrichtung 2 vermischt. Dazu wird die Rührwelle 7 mit den daran befindlichen Rührpaddeln 9 in der Funktionsstellung der Rührpaddel 9 um die Rührwelle 7 mittels dem Drehantrieb 8 in Pfeilrichtung eines Pfeiles 13 drehangetrieben. Die Rührwelle 7 ist dabei im unteren Bereich als Hohlwelle ausgeführt und über ein ortsfestes Führungsrohr 14, das am Boden des Fermenterbehälters 3 befestigt ist, gesteckt. Die Rührwelle 7 ist durch die Montageöffnung 6 in der Betondecke 5 des Fermenterbehälters 3 hinausgeführt, so dass der Drehantrieb 8 auf jeden Fall oberhalb des Flüssigkeitsspiegels der Fermenterflüssigkeit 4, bevorzugt wie im dargestellten Ausführungsbeispiel außerhalb des Fermenterbehälters 3 angeordnet ist. Die Montageöffnung 6 ist mit einer Manschette 15 gasdicht abgeschlossen, wobei die Rührwelle 7 ebenfalls gasdicht durch die Manschette 15 geführt ist. Die Rührpaddel 9 sind für eine optimale Durchmischung der Biomasse in der Fermenterflüssigkeit 4 axial und/oder radial gegeneinander versetzt an der Rührwelle 7 angeordnet und weisen in der Funktionsstellung einen bestimmten Anstellwinkel gegenüber der Rührwellenachse auf. Je nach eingesetzter Fermenterflüssigkeit 4 bzw. eingesetzter Biomasse kann die Länge der Rührpaddel 9 variieren, was mit einer strichlierten Verlängerung der beiden in Fig. 1 dargestellten Rührpaddel 9 schematisch eingezeichnet ist. Dabei können die Rührpaddel 9 mehrere Meter lang sein, wobei aber aufgrund der relativ geringen Drehgeschwindigkeit des Drehantriebes 8 trotz einer derartigen Länge der Rührpaddel 9 eine funktionssichere Drehbewegung der Rühreinrichtung 2 möglich ist. Für eine einfache Verdrehsicherung zwischen der Rührwelle 7 und dem Rührpaddel 9 ist die Rührwelle 7 wenigstens im Bereich der Rührpaddel 9 im Querschnitt gesehen quadratisch ausgeführt, was schematisch in Fig. 1 mit einer entsprechend hochgeklappten Schnittdarstellung des Querschnitts der Rührwelle 7 zwischen den beiden Rührpaddeln 9 eingezeichnet ist.

Soll für eine Wartung oder Reparatur der Rühreinrichtung 2 bzw. der Rührpaddel 9 die Rührwelle 7 mit den daran angeordneten Rührpaddeln 9 aus dem Fermenterbehälter 3 herausgezogen werden, so stellt sich aufgrund der in der Funktionsstellung befindlichen Rührpaddel 9 die Problematik, dass die, beispielsweise aus statischen Überlegungen heraus klein gehaltene Montageöffnung 6 für ein Herausziehen der Rührwelle 7 bei in der Funktionsstellung befindlichen Rührpaddeln 9 zu klein ist. Für eine Durchmesserverkleinerung der Anordnung Rührwelle 7 und Rührpaddel 9 können die Rührpaddel 9 von der Funktionsstellung in die Ausziehstellung um die Klappachse 12 geklappt werden. Dazu wird die Fixiereinrichtung 10 gelöst, so dass dadurch eine Klappung der Rührpaddel 9 freigegeben ist. In dem in Fig. 1 dargestellten Ausführungsbeispiel ist die Fixiereinrichtung 10 als lösbare Verriegelungsverbindung mit einer Schiebehülse 16 ausgeführt. Zur Verdeutlichung ist die Schiebehülse 16 des unteren Rührpaddels 9 geschnitten dargestellt. Zum Herausnehmen der Rührwelle 7 aus dem Fermenterbehälter 3 wird diese somit soweit nach oben gezogen, beispielsweise mit einer Kraneinrichtung, bis die Schiebehülse 16 des zu oberst angeordneten Rührpaddels 9 durch die Montageöffnung 6 erreichbar ist und für eine Freigabe der Klappung des Rührpaddels 9 hochgezogen wird (Pfeil 34). Für eine Vereinfachung dieses Hochziehens sind an der Schiebehülse 16 Einhakösen 17 vorgesehen, in die beispielsweise ein durch die Montageöffnung 6 eingeführter Haken eingehängt werden kann für ein für den ausführenden Werker vereinfachtes Hochziehen der Schiebehülse 16. Ebenso sind an den Rührpaddeln 9 Einhakösen 18 vorgesehen, in die für ein geführtes Klappen der Rührpaddel 9 zwischen der Funktionsstellung und der Ausziehstellung ebenfalls ein entsprechender Haken, der durch die Montageöffnung 6 in den Fermenterbehälter 3 geführt ist, eingehängt werden kann. Ist das oberste Rührpaddel 9 durch Hochziehen der Schiebehülse 16 in die Ausziehstellung umgeklappt, so kann die Rührwelle 7 bis zum nächsten Rührpaddel 9 weiter aus dem Fermenterbehälter 3 durch die Montageöffnung 6 hindurch herausgezogen werden, wobei nun beim nächsten Rührpaddel 9 ebenfalls ein Hochziehen der Schiebehülse 16 und somit ein Freigeben der Klappung des Rührpaddels 9 von der Funktionsstellung in die Ausziehstellung durchgeführt wird. Dies wird bei allen an der Rührwelle 7 angeordneten Rührpaddeln 9 durchgeführt, so dass die Rührwelle 7 mit entsprechend in die Ausziehstellung angeklappten Rührpaddeln 9 gänzlich aus dem Fermenterbehälter 3 herausgezogen werden kann. Nach etwaigen Wartungsarbeiten an der Rührwelle 7 bzw. an den Rührpaddeln 9 kann diese in umgekehrter Reihenfolge der Arbeitsschritte wieder in den Fermenterbehälter 3 eingebaut werden. Dazu wird die Rührwelle 7 jeweils so weit in den Fermenterbehälter 3 durch die Montageöffnung 6 hineingeführt, bis das erste Rührpaddel 9 innerhalb des Fermenterbehälters 3 angeordnet ist, so dass nun das Rührpaddel 9 von der Ausziehstellung in die entsprechende Funktionsstellung hochgeklappt wird und mittels der Schiebehülse 16, die hier als Fixiereinrichtung 10 vorgesehen ist, in der Funktionsstellung fixiert wird. Dies wird mit allen an der Rührwelle 7 angeordneten Rührpaddeln 9 der Reihe nach durchgeführt, bis die Rührwelle 7 gänzlich im Fermenterbehälter 3 auf dem Führungsrohr 14 aufgesteckt angeordnet ist.

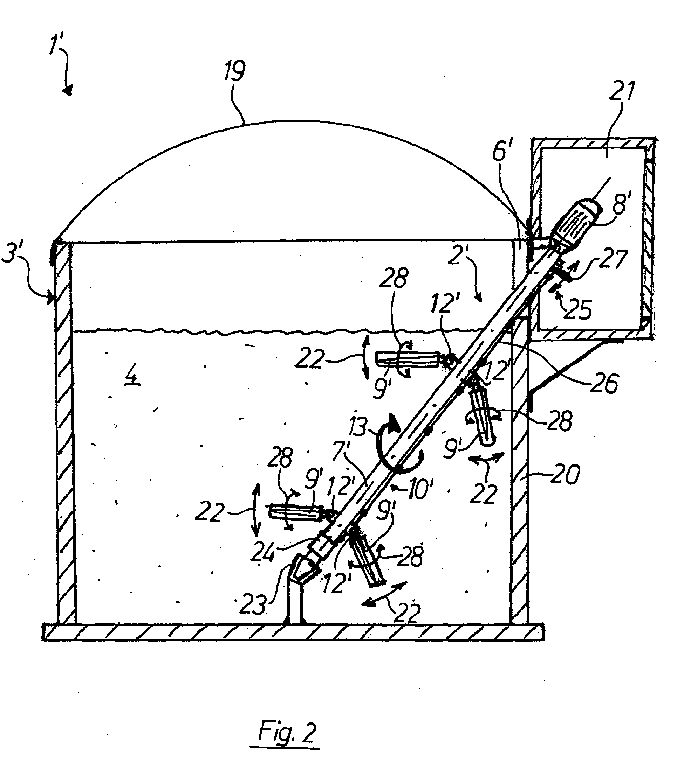

In Fig. 2 ist eine zweite Ausführungsform eines Fermenters 1' dargestellt, wobei Bauteile mit gleicher Funktion wie beim Fermenter 1 von Fig. 1 mit gleichen Bezugszeichen mit Strich versehen sind. Da der Fermenterbehälter 3' des Fermenters 1' ein Foliendach 19 aufweist, ist die Montageöffnung 6' im oberen Bereich einer Fermenterseitenwand 20 angeordnet. Die Rührwelle 7' ist von der Montageöffnung 6' ausgehend schräg nach unten in Richtung Boden des Fermenterbehälters 3' geführt. Am oberen Rührwellenende ist außerhalb des Fermenterbehälters 3' der Drehantrieb 8' angeordnet, wobei in dem in Fig. 2 dargestellten Ausführungsbeispiel des Fermenters 1' der Drehantrieb 8' in einem entsprechenden Serviceschacht 21 angeordnet ist. An der Rührwelle 7' sind jeweils gegenüberliegend auf gleicher Höhe die Rührpaddel 9' angeordnet, wobei auch hier eine Klappung der Rührpaddel 9' um die Klappachse 12', die in etwa senkrecht zur Rührwelle 7' ausgerichtet ist zwischen der Funktionsstellung und der Ausziehstellung der Rührpaddel möglich ist (Pfeil 22).

Am Fermenterboden ist eine ortsfest angebrachte Fangtasche 23 angeordnet, in die die Rührwelle 7' bei der Montage derselben in den Fermenterbehälter 3' eingesteckt ist. Ein Drehlager 24 ist versetzt zum unteren Rührenivellenende jedoch in dessen Nähe als Bestandteil der herausziehbaren Rührwelle 7' angebracht, so dass bei einem Herausziehen der Rührwelle 7' aus dem Fermenterbehälter 3' ebenfalls das Drehlager 24 mit herausgezogen wird, so dass etwaige Reparatur- bzw. Wartungsarbeiten auch am Drehlager 24 einfach durchgeführt werden können.

Die Fixiereinrichtung 10' ist beim Fermenter 1' durch eine außerhalb des Fermenterbehälters 3' betätigbare Stelleinrichtung 25 gebildet, mit der die Klappwinkel der einzelnen Rührpaddel 9' von der Funktionsstellung bis zur angeklappten Ausziehstellung verstellbar sind. Die Stelleinrichtung 25 weist eine Schubstange 26 auf, die entlang und außerhalb der Rührwelle 7' verläuft, wobei die Schubstange 26 mit den Rührpaddein 9' stellbeweglich verbunden ist. Die Schubstange 26 ist zusammen mit der Rührwelle 7' durch die Montageöffnung 6' in den Serviceschacht 21 geführt, wobei direkt benachbart zum Drehantrieb 8' innerhalb des Serviceschachtes 21 eine Handhabe 27 vorgesehen ist, mittels der eine Verstellung der Rührpaddel 9' durch eine Verschiebung der Schubstange 26 möglich ist. Grundsätzlich ist auch eine Verstellung des Anstellwinkels der Rührpaddel 9' mittels der Schubstange 26 denkbar (Pfeil 28).

Soll beim Fermenter 1' die Rührwelle 7' aus dem Fermenterbehälter 3' heraus und somit durch die Montageöffnung 6' hindurch demontiert werden, so wird mittels der Schubstange 26 eine Klappung der Rührpaddel 9' von der Funktionsstellung in die Ausziehstellung durchgeführt, so dass dadurch eine entsprechende Durchmesserverringerung der gesamten Anordnung erfolgt und diese somit problemlos durch die relativ kleine Montageöffnung 6' hindurch aus dem Fermenterbehälter 3' herausgezogen werden kann. Nach durchgeführter Wartung bzw. Reparatur beispielsweise eines Rührpaddels 9' wird die Rührwelle 7' mit in der Ausziehstellung befindlichen Rührpaddeln 9' durch die Montageöffnung 6' hindurch in den Fermenterbehälter 3' geführt, wobei das untere Rührwellenende entsprechend in die Fangtasche 23 eingesteckt wird.

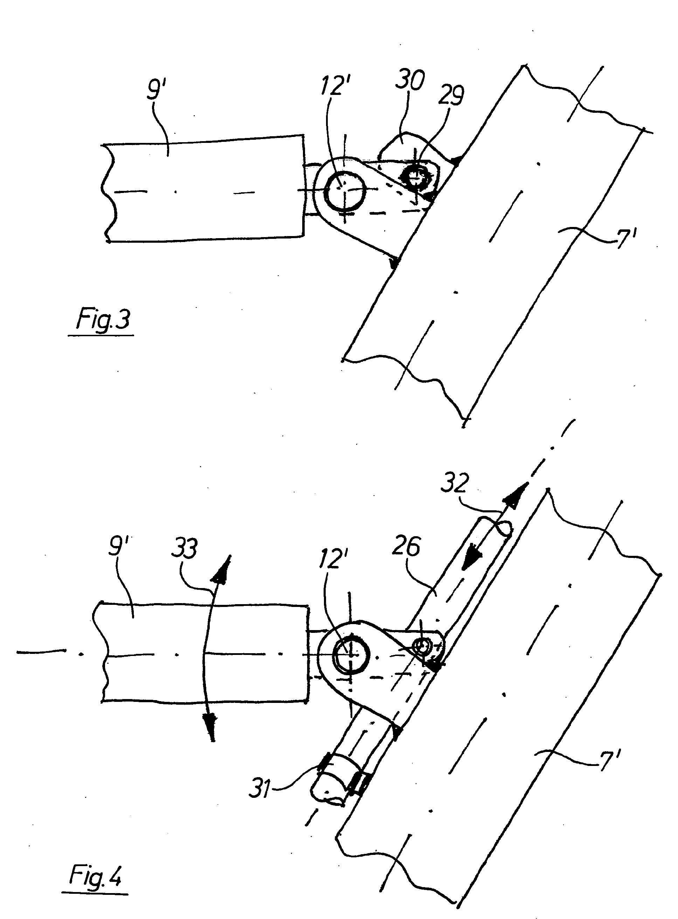

In Fig. 3 ist eine schematische Detailvergrößerung eines Teilbereichs der Rührwelle 7' von Fig. 2 gezeigt. Dabei ist die Klappachse 12' des Rührpaddels 9' zu erkennen, wobei das Rührpaddel 9' in der Funktionsstellung befindlich ist. Die Fixiereinrichtung 10' für die Halterung des Rührpaddels 9' in der Funktionsstellung ist hier durch einen lösbaren Bolzen 29 ausgeführt, der in einer an der Rührwelle 7' angeordneten Bolzenhalterung 30 eingesteckt ist. Bei der hier dargestellten Ausführungsvariante ohne Schubstange 26 ist eine für jedes Rührpaddel 9' separate Lösung des Bolzens 29 aus der Bolzenhalterung 30 notwendig, so dass nur ein schrittweises Herausziehen der Rührwelle 7' durch die Montageöffnung 6' möglich ist.

In Fig. 4 ist ebenfalls schematisch eine Detailvergrößerung eines Teilbereichs der Rührwelle 7' von Fig. 2 dargestellt, wobei hier eine Verstellung des Rührpaddels 9' mit der Schubstange 26 gezeigt ist. Die Schubstange 26 ist mit Halterungen 31 an der Rührwelle 7' verschiebbar befestigt. Bei einer Verschiebung der Schubstange 26 in Pfeilrichtung des Pfeiles 32 wird das Rührpaddel 9' entsprechend dem Pfeil 33 zwischen der hier dargestellten Funktionsstellung und der Ausziehstellung um die Klappachse 12' geklappt.

Unabhängig von der Ausführungsvariante des Fermenters 1 und 1' kann mit der erfindungsgemäßen Anordnung der Rührpaddel 9 und 9' an der Rührwelle 7 und 7' eine entsprechende Demontage der Rührwelle 7 durch Anklappen der Rührpadel 9 und 9' an die Rührwelle 7 und 7' stattfinden. Die dementsprechend zugeordnete Montageöffnung 6 und 6' kann dabei relativ klein ausgeführt werden, da aufgrund der Möglichkeit der Anklappung der Rührpaddel 9 und 9' eine entsprechende Durchmesserverkleinerung der gesamten Anordnung Rührpaddel/Rührwelle möglich ist.

## Patentansprüche

1. Fermenter einer Biogasanlage mit wenigstens einer Rühreinrichtung mit einer mittels eines Rührmotors drehangetriebenen und weit in den Fermenterbehälter ragenden Rührwelle und wenigstens einem an der Rührerwelle mitdrehend angebrachten Rührpaddel, welches unter die Flüssigkeitsoberfläche der vergärbaren Fermenterflüssigkeit mit Biomasse eintauchbar ist,
**dadurch gekennzeichnet,**
**dass** der Rührmotor (8; 8') an einem oberen Rührerwellenende angeschlossen ist und dieses in Funktionsstellung über die Flüssigkeitsoberfläche und aus einer Montageöffnung (6) in einer Fermenterdeckenwand (5) oder aus einer Montageöffnung (6') im oberen Bereich einer Fermenterseitenwand (20) herausragt,
**dass** das wenigstens eine Rührpaddel (9; 9')um eine etwa senkrecht zur Rührerwellenachse liegende, mit der Rührerwelle (7; 7') verbundene Achse (12; 12') klappbar verbunden ist und einerseits in eine von der Rührerwelle (7; 7') abragende Funktionsstellung aufklappbar und mittels einer Fixiereinrichtung (10; 10') fixierbar ist und andererseits für eine Durchmesserverkleinerung der Anordnung in eine Ausziehstellung für die Rührwelle (7; 7') an diese anklappbar ist, und
**dass** die Größe der Montageöffnung (6; 6') dem verkleinerten Durchmesser der Anordnung aus der Rührwelle (7; 7') und des daran angeklappten wenigstens einen Rührpaddels (9; 9') angepasst ist und die Anordnung in dieser Ausziehstellung für Wartungs- und/oder Reparaturzwecke aus dem Fermenter (1; 1') durch die Montageöffnung (6; 6') herausziehbar und demontierbar ist.

2. Fermenter nach Anspruch 1, **dadurch gekennzeichnet, dass** mehrere Rührpaddel (9; 9') axial und/oder radial gegeneinander versetzt an der Rührerwelle (7; 7') angeordnet sind.

3. Fermenter nach Anspruch 2, **dadurch gekennzeichnet, dass** jeweils zwei von mehreren Rührpaddeln (9') auf gleicher Höhe gegenüberliegend an der Rührerwelle (7') angeordnet sind.

4. Fermenter nach einem der Ansprüche 1 bis 3, **dadurch gekennzeichnet, dass** ein oder mehrere Rührpaddel (9; 9') als längliche Paddelplatten ausgebildet sind, mit einem in der Funktionsstellung bestimmten Anstellwinkel gegenüber der Rührerwellenachse.

5. Fermenter nach einem der Ansprüche 1 bis 4, **dadurch gekennzeichnet, dass** die Rührerwelle (7; 7')mit ihrem unteren Rührerende in eine am Fermenterboden ortsfest angebrachte Fangtasche (23) eingesteckt oder als Hohlwelle über ein ortsfestes Führungsrohr (14) gesteckt ist.

6. Fermenter nach Anspruch 5, **dadurch gekennzeichnet, dass** versetzt zum unteren Rührerwellenende jedoch in dessen Nähe ein Drehlager (24) als Bestandteil der herausziehbaren Rührerwelle (7') angebracht ist.

7. Fermenter nach einem der Ansprüche 1 bis 6, **dadurch gekennzeichnet, dass** die Rührerwelle (7; 7') bei einem Fermenter (1) mit einer stabilen Decke, insbesondere einer Betondecke (5) und darin enthaltener Montageöffnung (6) vertikal und bei einem Fermenter (1') mit Foliendach (19) und einer Montageöffnung (6') im oberen Bereich einer stabilen Seitenwand (20), insbesondere einer Betonseitenwand schräg nach unten angeordnet ist.

8. Fermenter nach einem der Ansprüche 1 bis 7, **dadurch gekennzeichnet, dass** das obere Rührerwellenende und/oder der Rührermotor in einen an die Montageöffnung (6') angeschlossenen gasdicht verschließbaren Serviceschacht (21) hineinragen und/oder dass das obere Rührerwellenende in der Montageöffnung (6) durch eine gasdicht abschließende Vorrichtung, insbesondere eine Manschette (15) oder Tauchtasse geführt ist.

9. Fermenter nach einem der Ansprüche 1 bis 8, **dadurch gekennzeichnet, dass** die Fixiereinrichtung (10; 10') für die Halterung des wenigstens einen Rührpaddels in der Funktionsstellung aus einer lösbaren Verriegelungsverbindung besteht, wobei der lösbare Riegel als Bolzen (29) und/oder Verschraubung und/oder Schiebehülse (16) ausgebildet ist.

10. Fermenter nach Anspruch 9, **dadurch gekennzeichnet, dass** die Verriegelungsverbindung wenigstens zwei Eingriffspositionen aufweist, so dass das zugeordnete Rührpaddel je nach den Gegebenheiten in einander wenigstens zwei zugeordneten unterschiedlichen Klappwinkein bezüglich der Rührerwellenachse in der Funktionsstellung fixierbar ist.

11. Fermenter nach einem der Ansprüche 1 bis 8, **dadurch gekennzeichnet, dass** die Fixiereinrichtung (10') für das wenigstens eine Rührpaddel (9') eine von außerhalb des Fermenters (1'), insbesondere im Bereich des oberen Rührerwellenendes betätigbare Stelleinrichtung (26) ist, mit der die Klappwinkel des Rührpaddels (9') von der Funktionsstellung bis zur angeklappten Ausziehstellung verstellbar sind.

12. Fermenter nach Anspruch 11, **dadurch gekennzeichnet, dass** die Stelleinrichtung aus wenigstens einer Schubstange (26) besteht, die entlang und innerhalb oder außerhalb der Rührerwelle (7') verläuft, wobei die Schubstange (26) mit wenigstens einem Rührpaddel (9'), vorzugsweise gekoppelt mit mehreren Rührpaddeln (9'), stellbeweglich verbunden und am oberen Rührerwellenendenbereich von Hand oder durch einen Schubstangen-Stellmotor betätigbar und in einer jeweils bestimmten Stellposition festlegbar ist.

13. Fermenter nach Anspruch 11, **dadurch gekennzeichnet, dass** die Stelleinrichtung aus einem unmittelbar an der Rührerwelle angeordneten und mit einem oder mehreren Rührpaddeln verbundenen hydraulischen und/oder elektrischen Rührpaddel-Stellmotor besteht.

14. Fermenter nach einem der Ansprüche 1 bis 13, **dadurch gekennzeichnet,**
**dass** das wenigstens eine Rührpaddel eine in Rührpaddellängsrichtung verlaufende Rührpaddelachse aufweist, auf der eine längliche Paddelplatte schwenkbar zur Veränderung des Anstellwinkels gelagert ist, und
**dass** zur Einstellung eines bestimmten Anstellwinkels eine Verriegelungseinrichtung mit mehreren Riegelpositionen vor Ort und/oder eine Fernbedienung mittels Schubstange und/oder Anstellwinkel-Stellmotor vorgesehen ist.

15. Fermenter nach einem Ansprüche 1 bis 14, **dadurch gekennzeichnet, dass** als Ergänzung zu der Rühreinrichtung mit Rührpaddeln zusätzlich wenigstens ein höhenverstellbares Tauchmotorrührwerk eingesetzt ist.
